# EUROPEAN PATENT APPLICATION

(11) **EP 4 835 510 A1**
(43) Date of publication of application: **04.11.2026**
(21) Application number: 26176086.2
(22) Date of filing: 01.05.2026
(51) Int. Cl.: G01R 33/563, A61B 5/055, G01R 33/56

(54) **MRI APPARATUS**

(30) Priority: 02.05.2025 US 202519196854
(71) Applicant: The Regents of the University of California, Oakland, CA 94607-5200 (US); Canon Kabushiki Kaisha, Tokyo 146-8501 (JP)
(72) Inventor: MIYAZAKI, Mitsue, Oakland, 94607-5200 (US); KASSAI, Yoshimori, Tokyo, 146-8501 (JP)
(74) Representative: Marks & Clerk LLP

(57) **Abstract**

In one embodiment, an MRI apparatus includes a setting unit configured to set a pulse sequence group that acquires first data and second data, the first date being for generating first perfusion data from behavior of a body fluid of an inflow path of an object included in a first imaging region, the second data being for generating second perfusion data from behavior of a body fluid of the inflow path included in a second imaging region located downstream of the first imaging region; a generation unit configured to generate the first perfusion data and the second perfusion data, both of which are obtained by applying the pulse sequence group to the object; and an evaluation unit configured to evaluate a disease status by comparing the first perfusion data with the second perfusion data.

## Description

### FIELD

Disclosed embodiments relate to a magnetic resonance imaging (MRI) apparatus.

### BACKGROUND

A fractional flow reserve (FFR) is known as a metric for evaluating the state of stenosis in the coronary artery. This FFR is a metric defined as the ratio of the internal pressure between the distal portion and proximal portion of the stenosis site, and measuring the FFR allows evaluation of the extent to which the blood flow is reduced at the stenosis site.

Conventionally known methods for obtaining the FFR includes an invasive examination using a catheter and/or guidewire. In this examination, after coronary artery angiography, a catheter and/or guidewire provided with a pressure sensor at its tip is inserted into the coronary artery to measure the internal pressure of the coronary artery, and the FFR is obtained by calculating the ratio of the internal pressure between the distal portion and the proximal portion of the stenosis site.

In another known method, the FFR is calculated by using the Navier-Stokes equation and the vascular shape depicted in a three-dimensional CT image, which is obtained by using an X-ray CT apparatus.

The examination using a pressure sensor for measuring the internal pressure of the coronary artery is invasive and imposes a burden on a patient that is the object. The examination using the X-ray CT apparatus involves exposure to radiation, and thus, imposes a burden on the patient as well.

An MRI apparatus is an imaging apparatus that excites nuclear spin of an object placed in a static magnetic field by applying a radio frequency (RF) signal having the Larmor frequency and reconstructs an image on the basis of magnetic resonance (MR) signals emitted from the object due to the excitation. Accordingly, an examination using an MRI apparatus is non-invasive without involving radiation exposure, and thus, is an examination that imposes less burden on the patient.

However, no method has been known to obtain the FFR from signals and/or image data acquired by using an MRI apparatus so far, regardless of whether it is contrast-enhanced imaging or non-contrast imaging. For this reason, there is a demand for a method in which a metric equivalent to or substitutable for the FFR can be acquired by using an MRI apparatus, which does not involve radiation exposure and does not require an invasive examination using a catheter and/or guidewire.

### SUMMARY OF THE INVENTION

One of the problems to be solved by the embodiments disclosed in the specification and drawings is to evaluate the presence or absence of a disease in an inflow path of an object without involving radiation exposure and without performing an invasive examination using a catheter and/or guidewire. It should be noted, however, that possible problems to be solved by the embodiments disclosed in the present specification and the drawings are not limited to the problem presented above. It is also possible to regard problems corresponding to the advantageous effects achieved by the configurations described in the following embodiments as other problems.

In one embodiment, an MRI apparatus includes a setting unit configured to set a pulse sequence group that acquires first data and second data, the first date being for generating first perfusion data from behavior of a body fluid of an inflow path of an object included in a first imaging region, the second data being for generating second perfusion data from behavior of a body fluid of the inflow path included in a second imaging region located downstream of the first imaging region; a generation unit configured to generate the first perfusion data and the second perfusion data, both of which are obtained by applying the pulse sequence group to the object; and an evaluation unit configured to evaluate a disease status by comparing the first perfusion data with the second perfusion data.

The inflow path may be an inflow blood vessel, the body fluid may be blood, and the evaluation unit may be further configured to; evaluate whether stenosis or occlusion has occurred between the first portion of the inflow path included in the first imaging region and the second portion of the inflow path included in the second imaging region by comparing the first perfusion data and the second perfusion data, both of which are generated by applying the second pulse sequence group to the object, or evaluate whether stenosis or occlusion has occurred between the second portion of the inflow path included in the second imaging region and a third portion of the inflow path included in the third imaging region by comparing the second perfusion data and the third perfusion data, both of which are generated by applying the second pulse sequence group to the object.

The setting unit may be further configured to set a second pulse sequence group that acquires; the first data for generating the first perfusion data from behavior of the body fluid of the inflow path of the object included in the first imaging region, the second data for generating the second perfusion data from behavior of the body fluid of the inflow path included in the second imaging region located downstream of the first imaging region, and third data for generating third perfusion data from behavior of the body fluid of the inflow path included in a third imaging region located further downstream of the second imaging region.

The evaluation unit may be further configured to; evaluate the disease status by comparing the first perfusion data and the second perfusion data, both of which are generated by applying the second pulse sequence group to the object, or evaluate the disease status by comparing the second perfusion data and the third perfusion data, both of which are generated by applying the second pulse sequence group to the object.

The evaluation unit may be further configured to; evaluate whether stenosis or occlusion has occurred between the first portion of the inflow path included in the first imaging region and the second portion of the inflow path included in the second imaging region by comparing the first perfusion data and the second perfusion data, both of which are generated by applying the second pulse sequence group to the object, or evaluate whether stenosis or occlusion has occurred between the second portion of the inflow path included in the second imaging region and a third portion of the inflow path included in the third imaging region by comparing the second perfusion data and the third perfusion data, both of which are generated by applying the second pulse sequence group to the object, wherein the inflow path is an inflow blood vessel, and the body fluid is blood.

Each of the pulse sequence group and the second pulse sequence group set by the setting unit may be a pulse sequence group in which behavior of body fluid of the inflow path is imaged without using a contrast agent.

The setting unit may be configured to set the pulse sequence group in such a manner that: each of the first imaging region and the second imaging region is provided approximately in parallel with a short-axis cross-section of a heart and has a predetermined thickness in a long-axis direction of the heart; and the first imaging region is set at a position closer to Basal than the second imaging region, wherein the inflow path is coronary artery.

The setting unit may be configured to set the second pulse sequence group in such a manner that: each of the first imaging region, the second imaging region, and the third imaging region is provided approximately in parallel with a short-axis cross-section of a heart and has a predetermined thickness in a long-axis direction of the heart; the first imaging region is set closer to Basal than the second imaging region; and the third imaging region is set closer to Apical than the second imaging region, wherein the inflow path is coronary artery.

Each of the first imaging region and the second imaging region corresponds to at least one segment that is obtained by dividing the heart in accordance with a Bull's Eye 17-segment model based on classification by American Heart Association (AHA), wherein the first imaging region and the second imaging region may be set in such a manner that: when the first imaging region is a segment in an outermost ring of three rings of the 17-segment model, the second imaging region is one segment in a central ring of the three rings and is a segment in a same radial direction as the first imaging region; or when the first imaging region is one segment in the central ring of the three rings of the 17-segment model, the second imaging region is one segment in an innermost ring of the three rings and is a segment in a same radial direction as the first imaging region.

The 17-segment model has a total of 17 segments composed of segments #1 to #17 classified by the AHA; wherein the first imaging region and the second imaging region may be set in such a manner that when the first imaging region is a segment #1, #2, #3, #4, #5, or #6, the second imaging region is a region corresponding to a segment #7, #8, #9, #10, #11, or #12, respectively.

The 17-segment model has a total of 17 segments composed of segments #1 to #17 classified by the AHA; wherein the first imaging region and the second imaging region may be set in such a manner that: when the first imaging region is a segment #7, the second imaging region a region corresponding to a segment #13; when the first imaging region is a segment #8, the second imaging region is a region corresponding to a segment #13 or #14; when the first imaging region is a segment #9, the second imaging region is a region corresponding to a segment #14 or #15; when the first imaging region is a segment #10, the second imaging region is a region corresponding to a segment #15; when the first imaging region is a segment #11, the second imaging region is a region corresponding to a segment #15 or #16; and when the first imaging region is a segment #12, the second imaging region is a region corresponding to a segment #16 or #13.

The MRI apparatus may be further include a display control unit configured to display at least one of perfusion-related metrics and perfusion curves in association with the at least one segment, wherein each of the first imaging region and the second imaging region corresponds to at least one segment that is obtained by dividing the heart in accordance with a Bull's Eye 17-segment model based on classification by American Heart Association (AHA).

The pulse sequence group may include a plurality of pulse sequences having a tag pulse for labeling body fluid; and each of the plurality of pulse sequences may be a pulse sequence group that acquires the first data of the inflow path in the first imaging region and the second data of the inflow path in the second imaging region by delaying a data acquisition timing from application of the tag pulse by a delay time TI that is set to be different for each of the plurality of pulse sequences.

The pulse sequence group may correspond to a time-SLIP Flow-Out method in which a tag pulse is applied to a tag region provided upstream of the first imaging region and then the first data and the second data are respectively acquired from the first imaging region and the second imaging region by delaying a data acquisition timing from application of the tag pulse by a plurality of different delay times TI; and the generation unit may be configured to generate the first perfusion data from acquired first data and generate the second perfusion data from acquired second data.

The pulse sequence group may correspond to a time-SLIP Flow-In method in which a tag pulse is applied to a region including the first imaging region and the second imaging region and then the first data and the second data are respectively acquired from the first imaging region and the second imaging region by delaying a data acquisition timing from application of the tag pulse by a plurality of different delay times TI; and the generation unit may be configured to generate the first perfusion data from acquired first data and generate the second perfusion data from acquired second data.

The pulse sequence group may be based on an electrocardiographic synchronization method and may acquire the first data and the second data in a same cardiac phase at different delay times TI, wherein the inflow path is coronary artery.

The pulse sequence group may acquire the first data and the second data in a same cardiac phase during diastole.

The generation unit may be configured to: calculate a first perfusion curve from the acquired first data as the first perfusion data; and calculate a second perfusion curve from the acquired second data as the second perfusion data.

The evaluation unit may evaluate the disease status by comparing a shape of the first perfusion curve with a shape of the second perfusion curve.

The MRI apparatus may further include a calculation unit configured to calculate a first metric and a second metric regarding perfusion from the first perfusion curve and the second perfusion curve, respectively, and the evaluation unit may configured to evaluate the disease status by using the first metric and the second metric.

The first metric may be at least one of peak height (PH) and time to peak (TTP) of the first perfusion curve. The second metric may be at least one of PH and TTP of the second perfusion curve.

When the second perfusion curve is lower in PH than the first perfusion curve or when TTP of the second perfusion curve is later than TTP of the first perfusion curve, the evaluation unit may evaluate that disease has occurred between the first portion of the inflow path included in the first imaging region and the second portion of the inflow path included in the second imaging region.

The MRI apparatus may further include a memory configured to store a lookup table or a trained model, the lookup table being a table that associates relationship between the first metric and the second metric with a fractional flow reserve (FFR) defined as a ratio of internal-pressure of the coronary artery as the inflow path between a distal portion and a proximal portion sandwiching a stenosis site of the coronary artery, the trained model being machine-learned to output the FFR from the relationship between the first metric and the second metric, wherein the evaluation unit may be configured to acquire the FFR from a relationship between the first metric and the second metric, by using the lookup table or the trained model.

The pulse sequence group set by the setting unit may be a pulse sequence group in which behavior of blood in the coronary artery is imaged by administering a contrast agent.

### BRIEF DESCRIPTION OF THE DRAWINGS

Fig. 1 is a configuration diagram illustrating an overall configuration of an MRI apparatus according to one embodiment.
Fig. 2 is a block diagram of the MRI apparatus according to the embodiment.
Fig. 3A and Fig. 3B are schematic diagrams illustrating the purpose of the MRI apparatus 1 according to the embodiment.
Fig. 4 is a flowchart illustrating operation of the MRI apparatus according to the embodiment.
Fig. 5A to Fig. 5C are schematic diagrams illustrating relationship between the first and second imaging regions and a 17-segment model.
Fig. 6A to Fig. 6F are schematic diagrams illustrating the 17-segment model.
Fig. 7A to Fig. 7C are schematic diagrams illustrating an operation concept of a Time-SLIP flow-out method.
Fig. 8A to Fig. 8E are schematic diagrams illustrating a pulse sequence group of the flow-out method.
Fig. 9A to Fig. 9C are schematic diagrams illustrating an operation concept of a Time-SLIP flow-in method.
Fig. 10A to Fig. 10E are schematic diagrams illustrating a pulse sequence group of the flow-in method.
Fig. 11A to Fig. 11C are schematic diagrams illustrating a processing concept from the step ST101 to the step ST103 in Fig. 4.
Fig. 12A and Fig. 12B are schematic diagrams illustrating shapes of first and second perfusion curves when an abnormal site such as stenosis and occlusion exists.
Fig. 13A to Fig. 13D are schematic diagrams illustrating the first and second perfusion curves associated with respective segments of the outermost and central rings of the 17-segment model.
Fig. 14A to Fig. 14D are schematic diagrams illustrating the first and second perfusion curves associated with respective segments of the central and innermost rings of the 17-segment model.
Fig. 15A to Fig. 15D are schematic diagrams illustrating a method of evaluating presence/absence of an abnormality such as stenosis and occlusion by using another evaluation metric.
Fig. 16A to Fig. 16C are schematic diagrams illustrating a processing concept of the step ST105 in Fig. 4.

### DETAILED DESCRIPTION

Hereinbelow, embodiments of an MRI apparatus 1 will be described by referring to the accompanying drawings.

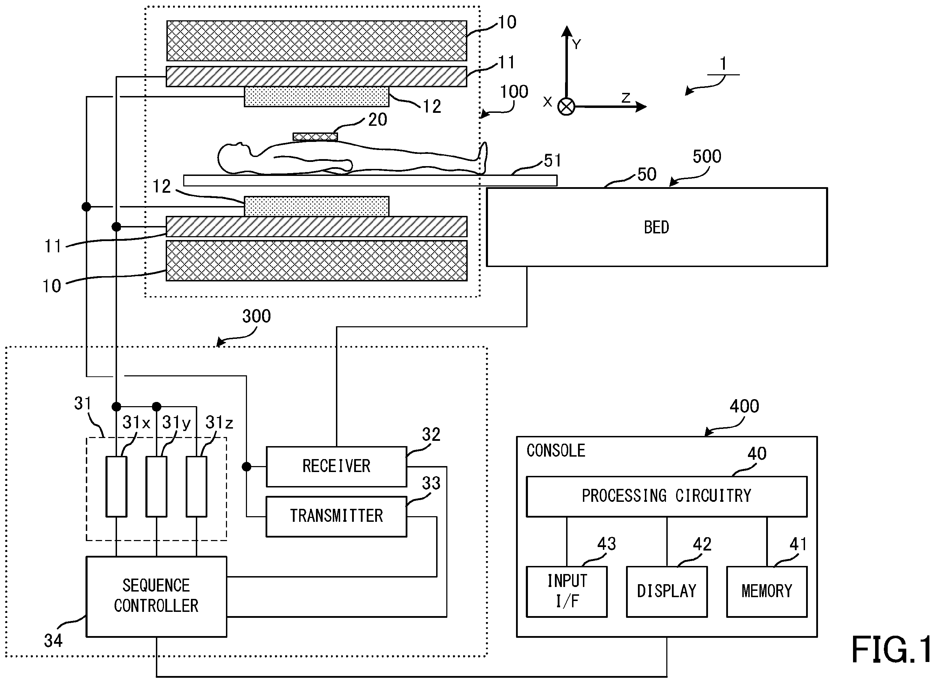

Fig. 1 is a block diagram illustrating an overall configuration of the MRI apparatus 1 according to one embodiment. The MRI apparatus 1 includes a gantry 100, a control cabinet 300, a console 400, and a bed 500, for example.

The gantry 100 includes a static magnetic field magnet 10, a gradient coil 11, and a whole body (WB) coil 12, and these components are housed in a cylindrical housing. The bed 500 includes a bed body 50 and a table 51. Further, the MRI apparatus 1 includes an array coil 20 to be disposed close to an object.

The control cabinet 300 includes three gradient-coil power supplies 31 (31x for the X-axis, 31y for the Y-axis, and 31z for the Z-axis), an RF receiver 32, an RF transmitter 33, and a sequence controller 34.

The static magnetic field magnet 10 of the gantry 100 is substantially in the form of a cylinder, and generates a static magnetic field inside a bore, which is an imaging space of an object such as a patient (i.e., a space inside the cylindrical structure of the static magnetic field magnet 10). The static magnetic field magnet 10 includes a superconducting coil inside, and the superconducting coil is cooled down to an extremely low temperature by liquid helium. The static magnetic field magnet 10 generates a static magnetic field by supplying the superconducting coil with an electric current provided from a static-magnetic-field power supply (not shown) in an excitation mode. Afterward, the static magnetic field magnet 10 shifts to a persistent current mode, and the static-magnetic-field supply is disconnected. Once it enters the persistent current mode, the static magnetic field magnet 10 continues to generate a strong static magnetic field for a long time, for example, over one year. Note that the static magnetic field magnet 10 may be configured as a permanent magnet.

The gradient coil 11 is also substantially in the form of a cylinder and is fixed to the inside of the static magnetic field magnet 10. This gradient coil 11 applies gradient magnetic fields to the object in the respective directions of the X-axis, the Y-axis, and the Z-axis by being supplied with electric currents from the respective gradient-coil power supplies 31x, 31y, and 31z.

The bed body 50 of the bed 500 can move the table 51 in the vertical direction and in the horizontal direction, and moves the table 51 with the object placed thereon to a predetermined height before imaging. Afterward, when the object is imaged, the bed body 50 moves the table 51 in the horizontal direction so as to move the object to the inside of the bore.

The WB body coil 12 is shaped substantially in the form of a cylinder so as to surround the object, and is fixed to the inside of the gradient coil 11. The WB coil 12 applies RF pulses transmitted from the RF transmitter 33 to the object, and receives MR signals emitted from the object due to excitation of hydrogen nuclei.

The array coil 20 is an RF coil that receives MR signals emitted from the object at a position close to the object. The array coil 20 is composed of a plurality of coil elements, for example. There are various models for the array coil 20 depending on an anatomical imaging part of the object, such as for the head, for the chest, for the spine, for the legs, and for the whole body. Fig. 1 illustrates an array coil 20 for the chest.

The RF transmitter 33 transmits an RF pulse to the WB coil 12 on the basis of an instruction from the sequence controller 34.

The RF receiver 32 detects the MR signals received by the WB coil 12 and/or the array coil 20, and transmits raw data obtained by digitizing the detected MR signals to the sequence controller 34.

Under the control of the console 400, the sequence controller 34 performs a scan of the object by driving the gradient-coil power supplies 31, the RF transmitter 33, and the RF receiver 32. When the sequence controller 34 receives the raw data from the RF receiver 32 by performing the scan, the sequence controller 34 transmits the raw data to the console 400.

The sequence controller 34 includes processing circuitry (not shown). This processing circuitry is composed of hardware such as a processor configured to execute predetermined programs, a field programmable gate array (FPGA), and an application specific integrated circuit (ASIC).

The console 400 is configured as a computer that includes processing circuitry 40, a memory 41, a display 42, and an input I/F (interface) 43.

The memory 41 is a recording medium including a read-only memory (ROM) and a random access memory (RAM) in addition to an external memory device such as a hard disk drive (HDD) and an optical disc device. The memory 41 stores various programs to be executed by the processor of the processing circuitry 40 as well as various data and information.

The input I/F 43 includes various devices for a user to input various data and information, such as a mouse, a keyboard, a trackball, and a touch panel.

The display 42 is a display device such as a liquid crystal display panel, a plasma display panel, and an organic EL panel.

The processing circuitry 40 is, for example, a circuit provided with a central processing unit (CPU) and/or a special-purpose or general-purpose processor. The processor implements various functions described below by executing the programs stored in the memory 41. The processing circuitry 40 may be configured as hardware such as an FPGA and an ASIC. The various functions described below can also be implemented by such hardware. Additionally, the processing circuitry 40 can implement the various functions by combining hardware processing and software processing based on its processor and programs.

The console 400 controls the entirety of the MRI apparatus 1 by using these components. Specifically, the console 400 receives imaging conditions and other various instructions and information entered by a user such as a medical imaging technologist via the input I/F 43 including a mouse and a keyboard. The processing circuitry 40 causes the sequence controller 34 to execute a scan on the basis of the inputted imaging conditions, and reconstructs an image on the basis of the raw data transmitted from the sequence controller 34. The reconstructed image is displayed on the display 42 or stored in the memory 41.

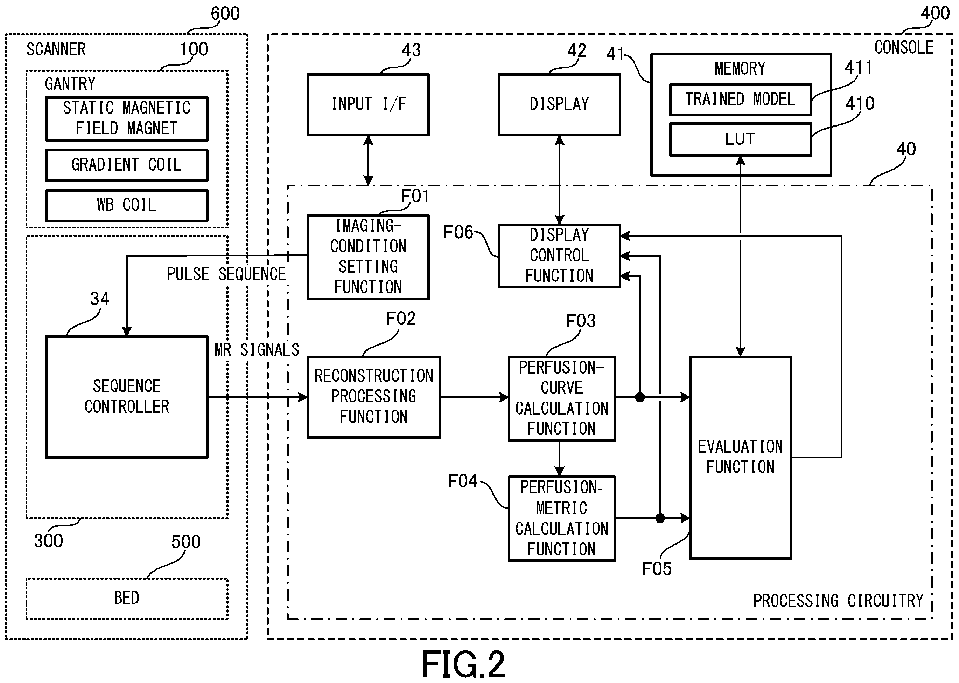

Fig. 2 is a block diagram illustrating the MRI apparatus 1 according to the embodiment, particularly focusing on the functions to be implemented by the processing circuitry 40 of the console 400. Among the components of the MRI apparatus 1 shown in Fig. 1, the components excluding the console 400, i.e., the control cabinet 300, the gantry 100, and the bed 500, are collectively referred to as a scanner 600 in Fig. 2.

The processing circuitry 40 implements each of an imaging-condition setting function F01, a reconstruction processing function F02, a perfusion-curve calculation function F03, a perfusion-metric calculation function F04, an evaluation function F05, and a display control function F06.

The imaging-condition setting function F01 sets a pulse sequence group that acquires the first data and the second data. The first data are used for generating the first perfusion data from behavior of the blood flow of the coronary artery of the object included in the first imaging region The second data are used for generating the second perfusion data from the behavior of the blood flow in the coronary artery included in the second imaging region that is located on the downstream side of the first imaging region. Specific example of the pulse sequence group to be set by the imaging-condition setting function F01 will be described below.

The reconstruction processing function F02 acquires MR signals emitted from the object by applying the pulse sequence group to the object, and reconstructs a plurality of time-sequential MR images from the acquired MR signals.

The perfusion-curve calculation function F03 calculates the first perfusion curve as the first perfusion data and the second perfusion curve as the second perfusion data, by using signal intensity (i.e., pixel values or voxel values) at specific voxel positions of the plurality of time-sequential MR images.

The perfusion-metric calculation function F04 calculates the first metrics as the first perfusion data from the first perfusion curve, and calculates the second metrics as the second perfusion data from the second perfusion curve.

The evaluation function F05 compares the first perfusion data (i.e., at least one of the first perfusion curve and the first metrics, in other words, the perfusion curve and/or the perfusion metrics) with the second perfusion data (i.e., at least one of the second perfusion curve and the second metrics, in other words, the second perfusion and/or the second metrics) so as to evaluate occurrence/non-occurrence of stenosis or occlusion between the first portion of the coronary artery included in the first imaging region and the second portion of the coronary artery included in the second imaging region. In this evaluation, the evaluation function F05 can refer to a lookup table (LUT) 410 stored in the memory 41 and can use a trained model 411 stored in the memory 41.

The display control function F06 performs processing of causing the display 42 to display at least one of the following: (a) the calculated first and second perfusion curves; (b) the first and second metrics; (c) metrics indicating the relationship between the first metrics and the second metrics; and (d) the evaluation result obtained by the evaluation function F05.

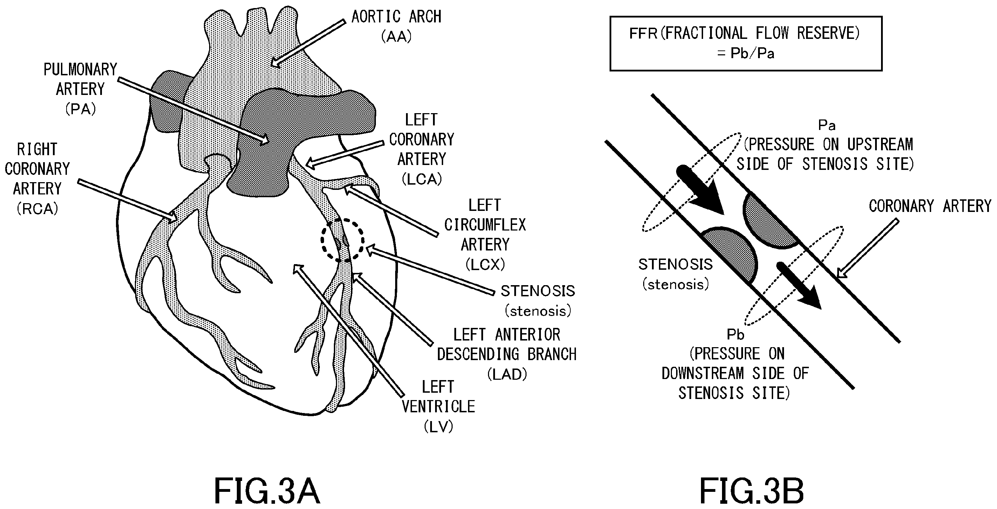

The specific processing and operation of each of the above-described functions will be described in detail by using Fig. 3A to Fig. 16C.

Fig. 3A and Fig. 3B illustrate the purpose of the MRI apparatus 1 according to the embodiment. The imaging target of the MRI apparatus 1 according to the embodiment is mainly the blood flow in the heart and/or the brain as shown in Fig. 3A and Fig. 3B. In particular, when the imaging target is the heart, the imaging target is the behavior of the blood flow in the coronary artery. Hereinbelow, a description will be given of a case where the imaging target is the coronary artery of the heart.

As shown in Fig. 3A, the coronary artery is broadly divided into the right coronary artery (RCA) and the left coronary artery (LCA), and the left coronary artery is further divided into the left anterior descending artery (LAD) and the left circumflex artery (LCX).

"Stenosis" is a condition in which the inner diameter of these vessels constituting the coronary artery narrows for some reason. When stenosis occurs, the heart muscles cannot receive enough blood, resulting in symptoms such as angina pectoris. On the other hand, "occlusion" is a phenomenon in which stenosis progresses to a more advanced stage and the blood flow stops in this progressed region of stenosis. If occlusion of the coronary artery occurs, it leads to a serious condition such as myocardial infarction.

Therefore, it is important to detect stenosis at an early stage before leading to occlusion and to measure the degree of stenosis. A fractional flow reserve (FFR) is known as a metric indicative of the degree of stenosis. As shown in Fig. 3B, the FFR is a metric defined as a ratio of the internal pressure Pb of the distal (or downstream) portion of the coronary artery in the stenosis site with respect to the internal pressure Pa of the proximal (or upstream) portion of the coronary artery in the stenosis site (FFR = Pb/Pa).

As described above, known methods for obtaining the FFR include: a method in which a catheter and/or guidewire provided with a pressure sensor at its tip is inserted into the coronary artery to measure the internal pressure of the coronary artery; and another method of calculating the FFR by using the Navier-Stokes equation and the vascular shape depicted in a three-dimensional CT image generated by an X-ray CT apparatus.

However, the examination using the pressure sensor for measuring the internal pressure of the coronary artery is invasive and the examination using the X-ray CT apparatus involves exposure to radiation, and thus, both methods impose a burden on the patient to a certain degree.

Hence, the MRI apparatus 1 according to the present embodiment is configured to be able to non-invasively measure presence/absence of stenosis and/or occlusion in the coronary artery and the degree of stenosis without radiation exposure, as described below.

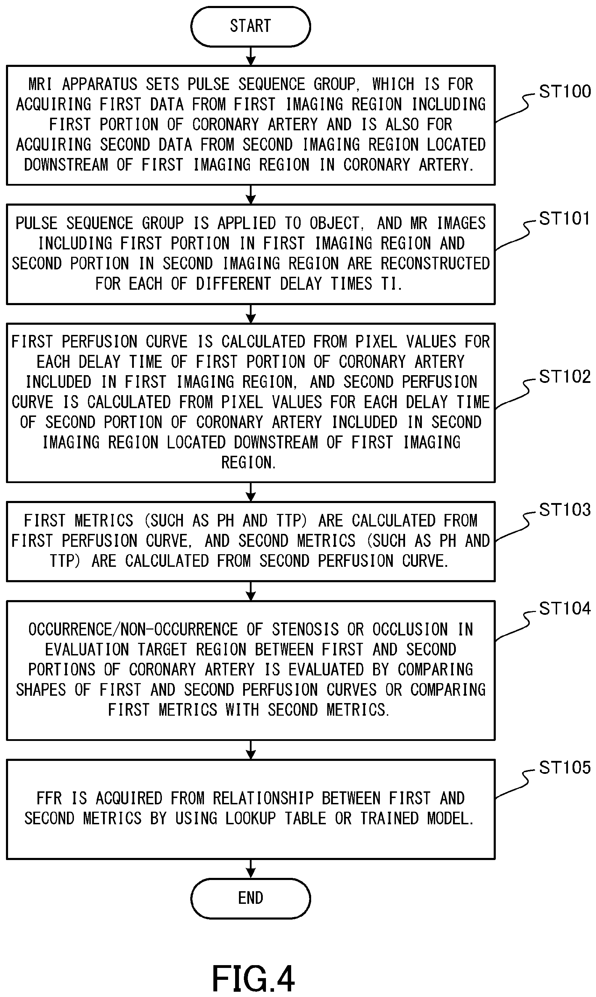

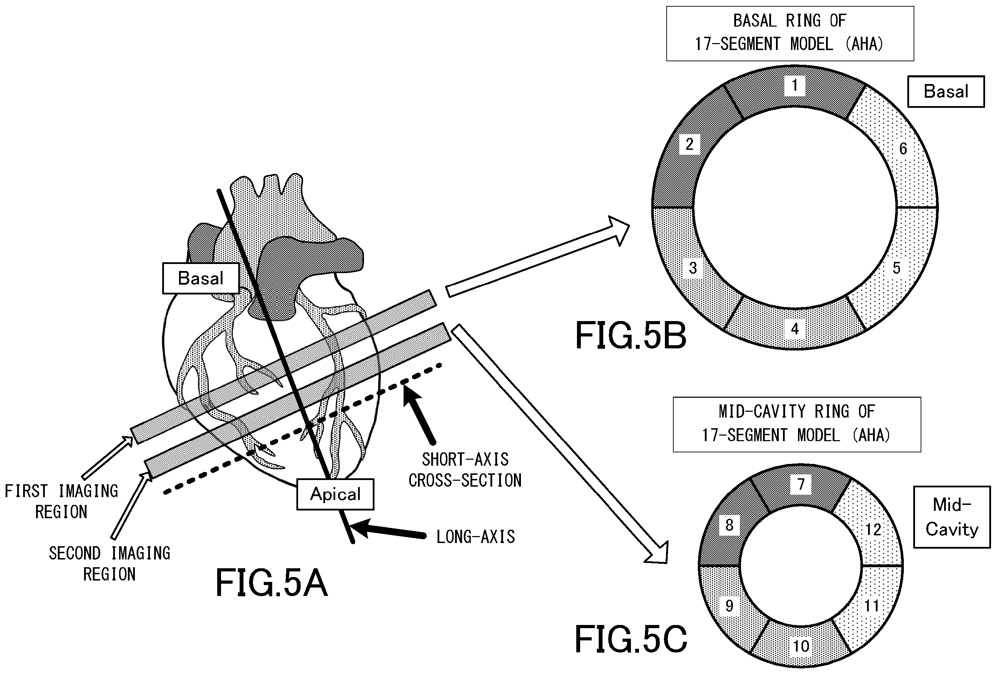

Fig. 4 is a flowchart illustrating the operation of the MRI apparatus 1. Hereinbelow, the operation of the MRI apparatus 1 will be described in detail based on the processing flow shown in the flowchart of Fig. 4 by referring to schematic diagrams of Fig. 5A to Fig. 16C as required.

First, in the step ST100 of Fig. 4, the MRI apparatus 1 sets a pulse sequence group, which is for acquiring the first data from the first imaging region including the first portion of the coronary artery and is also for acquiring the second data from the second imaging region located downstream of the first imaging region in the the coronary artery.

Fig. 5A schematically illustrates the positions and sizes of the first and second imaging regions. The first imaging region is set to be located more upstream of the coronary artery than the second imaging region.

As is well known, the main flow of blood in each of the coronary arteries including the right coronary artery (RCA), the left anterior descending artery (LAD), and the left circumflex artery (LCX) is from Basal (i.e., basal segment) to Apical (i.e., Apical segment). Thus, the processing circuitry 40 of the MRI apparatus 1 sets the pulse sequence group in such a manner that: (a) each of the first and second imaging regions is provided approximately in parallel with the short-axis cross-section of the heart and has a predetermined thickness in the long-axis direction of the heart; and (b) the first imaging region is closer to Basal of the heart than the second imaging region. This setting allows the first imaging region located more upstream of the coronary artery than the second imaging region.

A model that classifies the location of the coronary artery by a ring model within three short-axis cross-sections between Basal and Apical is known as a 17-segment model. The 17-segment model is a model defined by the American Heart Association (AHA) and is sometimes called a bull's eye 17-segment model.

When the 17-segment model is applied to the first and second imaging regions, for example, the first imaging region can be associated with the ring on Basal shown in Fig. 5B, and the second imaging region can be associated with the ring of Mid-Cavity (i.e., mid-cavity segment) shown in Fig. 5C.

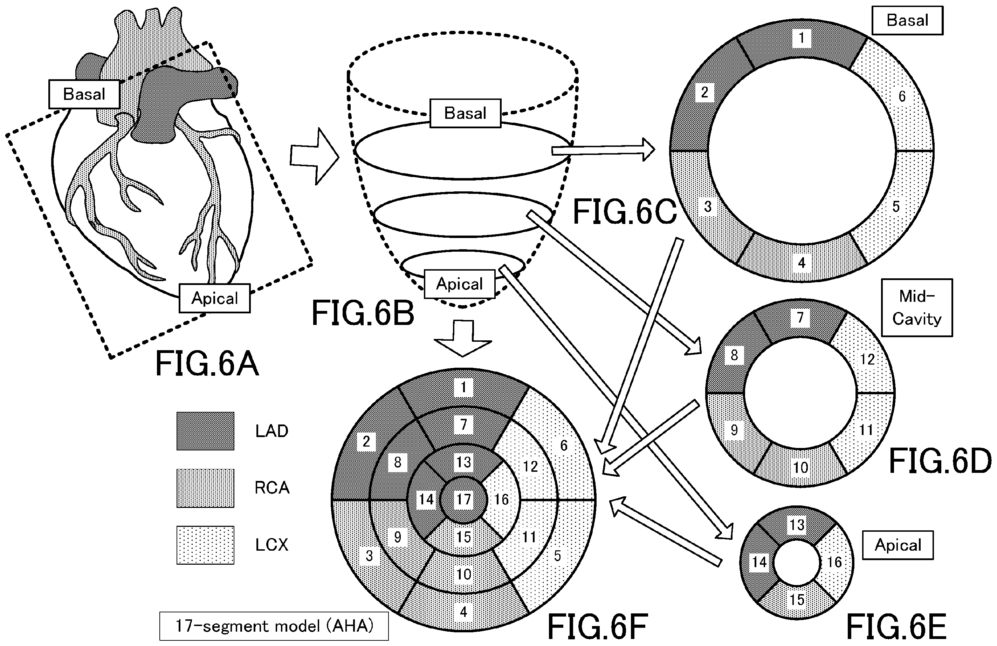

Alternatively, the first imaging region can be associated with the ring of Mid-Cavity and the second imaging region can be associated with the ring on Apical shown in Fig. 6E.

As long as the first imaging region is located more upstream of the coronary artery than the second imaging region as the above-described condition, the first and second imaging regions may be set to any desired size at any desired position without depending on the 17-segment model.

For example, both may be set such that the first imaging region is on the upstream side (closer to Basal) inside the ring of Mid-Cavity and the second imaging region is on the downstream side (closer to Apical) inside the ring of the same Mid-cavity.

Fig. 6A to Fig. 6F illustrate the 17-segment model more specifically. In the 17-segment model as shown in Fig. 6A and Fig. 6B, the shape of the heart is modeled as a shape obtained by dividing a prolate ellipsoid into two at the center in the long axis direction, and the circumference of this semi-prolate ellipsoid shape is further classified into three rings: Basal ring on shown in Fig. 6C, Mid-Cavity ring shown in Fig. 6D, and Apical ring shown in Fig. 6E.

The ring on Basal (or The Basal ring) is divided into six segments #1 to #6, and the ring in the central portion is divided into six segments #7 to #12. The ring on Apical is divided into four segments #13 to #17.

In Fig. 6F, these three rings and the circle corresponding to the tip of the semi-prolate ellipsoid (i.e., the segment #17) are arranged coaxially, and this Fig. 6F defines the 17-segment model. The 17-segment model divides the outer circumferential region of the heart into 17 segments from the segments #1 to #17, and defines the segment(s) corresponding to each of the right coronary artery (RCA), the left anterior descending artery (LAD), and the left circumflex artery (LCX) which are the major coronary arteries.

Specifically, it defines that each of the segments #1, #2, #7, #8, #13, #14, and #17 corresponds to the left anterior descending artery (LAD), each of the segments #3, #4, #9, #10, and #15 corresponds to the right coronary artery (RCA), and each of the segments #5, #6, #11, #12, and #16 correspond to the left circumflex artery (LCX).

As shown in Fig. 5A, the first and second imaging regions are cross-sections set approximately in parallel with the short-axis cross-section of the heart. In each of these cross-sections, the region including the upstream portion (i.e., the first portion) of the specific coronary artery to be examined may be referred as the first imaging region, and the region including the downstream portion (i.e., the second portion) of the specific coronary artery may be referred as the second imaging region. Here, the specific coronary artery is, for example, one of the right coronary artery (RCA), the left anterior descending artery (LAD), and the left circumflex artery (LCX), which are the major coronary arteries.

When the first and second imaging regions are defined so as to correspond to a specific coronary artery as described above, the first and second imaging regions and each segment of the 17-segment model can be defined in several positional relationships as follows by referring to the chart of the 17-segment model shown in Fig. 6F. In other words, as shown below, when the first imaging region is set so as to correspond to the upstream segment of a specific coronary artery, the positional relationship between the first and second imaging regions and each segment can be defined such that the second imaging region corresponds to the downstream segment of the specific coronary artery.

Firstly, it can be defined that: (a) when the first imaging region is one segment in the outermost ring of the three rings in the 17-segment model, the second imaging region is one segment in the central ring of the three rings and also is the segment in the same radial direction as the first imaging region; or (b) when the first imaging region is one segment in the central ring of the three segments in the 17-segment model, the second imaging region is one segment in the innermost ring of the three rings and also is the segment in the same radial direction as the first imaging region.

Secondly, it can be defined that when the first imaging region is the segment #1, #2, #3, #4, #5, or #6, the second imaging region is the region corresponding to the segment #7, #8, #9, #10, #11, or #12, respectively.

Thirdly, it can be defined that: when the first imaging region is the segment #7, the second imaging region corresponds to the segment #13; when the first imaging region is the segment #8, the second imaging region corresponds to the segment #13 or #14; when the first imaging region is the segment #9, the second imaging region corresponds to the segment #14 or #15; when the first imaging region is the segment #10, the second imaging region corresponds to the segment #15; when the first imaging region is the segment #11, the second imaging region corresponds to the segment #15 or #16; and when the first imaging region is the segment #12, the second imaging region corresponds to the segment #16 or #13.

So far, a description has been given of imaging regions (i.e., the first and second imaging regions) of the pulse sequence group set in the step ST100. Next, a description will be given of the type of the pulse sequence group (or the sequence diagram of the pulse sequence group) set in the step ST100.

The pulse sequence group set by the imaging-condition setting function F01 is mainly the pulse sequence group for non-contrast MRA (MR Angiography) in which blood behavior of the coronary artery is imaged without using a contrast agent and is not an imaging method of using a contrast agent, and thus, perfusion information for the first imaging region and perfusion information for the second imaging region can be measured independently, which is a feature of this method. Additionally or alternatively, it may be the pulse sequence group of contrast-enhanced MRA (perfusion MRI) in which the respective short-axis cross-sections of the above-described three regions including Basal, Mid-Cavity, and Apical are simultaneously imaged as the state of the coronary artery by using a contrast agent with high time resolution.

The pulse sequence group for non-contrast MRA is composed of a plurality of pulse sequences, which individually include application of a tag pulse for labeling blood and are different from each other in delay time TI from application of the tag pulse to a data acquisition timing. Specifically, these pulse sequences as a whole acquire the first data of the coronary artery in the first imaging region and the second data of the coronary artery in the second imaging region for each of the different delay times TI.

The first metrics and/or the first perfusion curve are calculated as the first perfusion data from the plurality of first data that are acquired for the plurality of delay times TI, as described below. Similarly, the second metrics and/or the second perfusion curve are calculated as the second perfusion data from the plurality of second data that are acquired for the plurality of delay times TI.

On the other hand, in the pulse sequence group of contrast-enhanced MRA, after a contrast agent is administered into a blood vessel such as a vein and the contrast agent reaches the heart, an imaging sequence without a tag pulse is performed to acquire: a plurality of first data of the coronary artery in the first imaging region; a plurality of second data of the coronary artery in the second imaging region; and, if necessary, a plurality of third data of the coronary artery in the third imaging region (including the segments #13, #14, #15, and #16 in the 17-segment model), at a predetermined time intervals. Further, the first metrics and/or the first perfusion curve are calculated as the first perfusion data from the plurality of first data, the second metrics and/or the second perfusion curve are calculated as the second perfusion data from the plurality of second data, and the third metrics and/or the third perfusion curve are calculated as the third perfusion data from the plurality of third data, similarly to the above-described non-contrast MRA.

Hereinbelow, a description will be given of the pulse sequence group for the non-contrast MRA. Although there are several types of pulse sequence groups for the non-contrast MRA, the pulse sequence group for the non-contrast MRA used in the MRI apparatus 1 of the embodiment is not limited to a specific type as long as the pulse sequence group includes a tag pulse for labeling blood.

For example, the pulse sequence group may be a group based on an ASL (Arterial Spin Labeling) method in which a difference between tag-on data acquired by a tag-on sequence having a tag pulse and tag-off data acquired by a tag-off sequence without including a tag-on pulse is calculated. A pulse sequence group based on a Time-SLIP (Time-Spatial Labeling Inversion Pulse) method, which is a type of the ASL method, may also be used. The Time-SLIP method is further classified into a Flow-Out method and a Flow-In method.

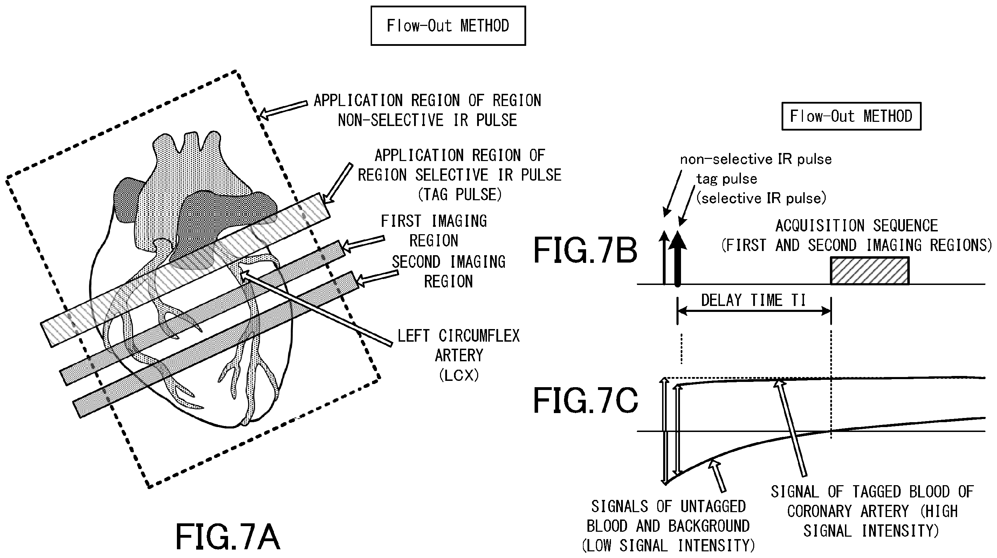

Fig. 7A to Fig. 7C illustrate the operation concept of the Flow-Out method. In the Flow-Out pulse sequence as shown in Fig. 7B, a region non-selective IR pulse is applied, and subsequently, a region-selective IR pulse (i.e., tag pulse) is applied. After elapse of a delay time TI from application of the tag pulse, an acquisition sequence is applied to acquire MR signals from the first and second imaging regions.

The region non-selective IR pulse is applied to the region including the entire heart as shown in the dashed quadrangular frame in Fig. 7A, and the longitudinal magnetization of the entirety of this region is inverted as shown in Fig. 7C. The tag pulse is applied to the region further upstream of the first imaging region so as to further invert the longitudinal magnetization of the blood in the coronary artery in this tag-pulse application region (i.e., the tag region), and consequently, the blood in the coronary artery in the region upstream of the first imaging region is tagged (or labeled).

As shown in Fig. 7C, the blood in the tagged coronary artery is higher in signal intensity than the untagged blood and the background such as the myocardium. After elapse of the delay time TI, the blood in the coronary artery with its increased signal intensity flows into the first and second imaging regions, and MR signals are acquired by a predetermined acquisition sequence.

The pulse sequence group repeats the pulse sequence shown in Fig. 7B with different delay times TI. The shape of the heart changes depending on heartbeat. Hence, each pulse sequence of the pulse sequence group is required to acquire the first and second data from the first and second imaging regions during the same cardiac phase while differentiating the delay times TI of these pulse sequences from each other. Thus, in the MRI apparatus 1 of the embodiment, it is necessary to use the pulse sequence group based on an electrocardiographic synchronization method.

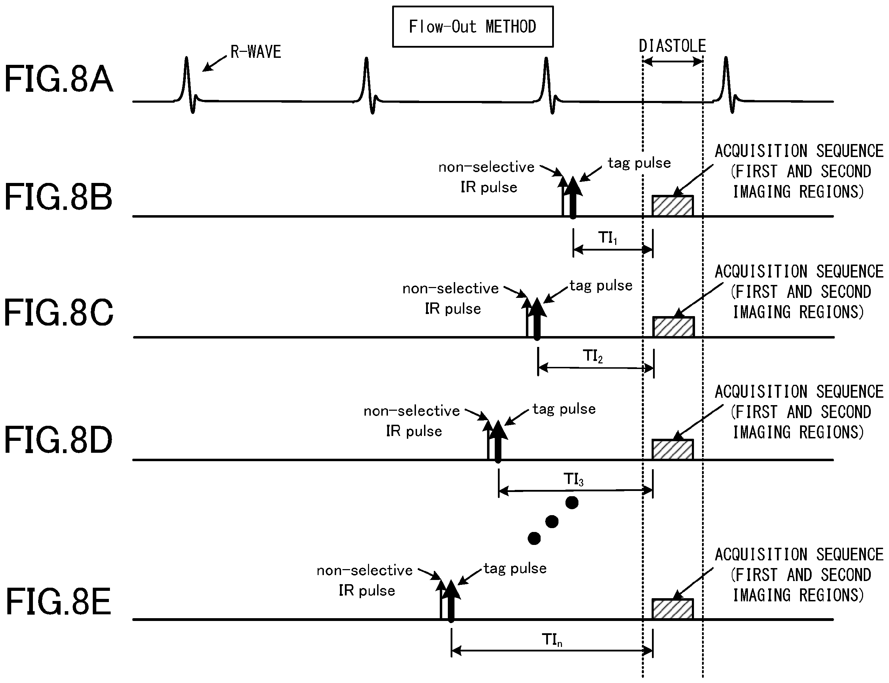

Fig. 8A to Fig. 8E illustrate a pulse sequence group of the Flow-Out method based on electrocardiographic synchronization imaging.

Fig. 8A shows an ECG signal for electrocardiographic synchronization, and Fig. 8B to Fig. 8E show respective sequence diagrams of the Flow-Out method with different delay times TI. In the case shown in Fig. 8B to Fig. 8E, each acquisition sequence is started at the same cardiac phase during diastole.

The acquisition sequence may begin in diastole or in systole as long as each acquisition sequence begins at the same cardiac phase.. However, in consideration the fact that the rate of temporal change in the heart shape is smaller during diastole than during systole and the period of the cardiac cycle is usually changes, it is preferred to set the acquisition sequence during diastole rather than during systole.

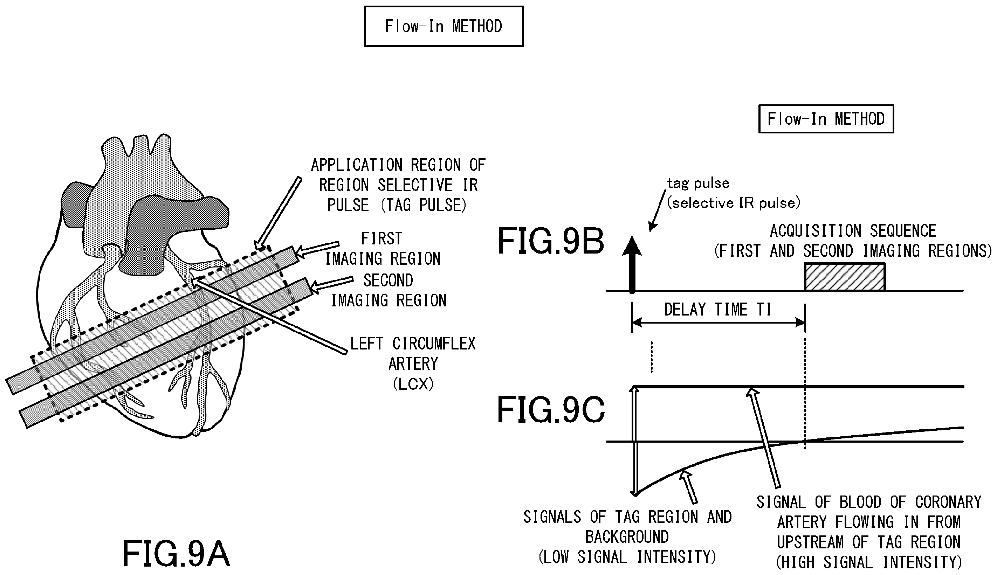

On the other hand, Fig. 9A to Fig. 9C illustrate the operation concept of the Flow-In method. In the Flow-In pulse sequence, any region non-selective IR pulse is not applied as shown in Fig. 9B, and only the region selective IR pulse (i.e., tag pulse) is applied, and after elapse of the delay time TI from application of the tag pulse, the acquisition sequence is applied to acquire MR signals from the first and second imaging regions.

The tag pulse is applied to the region (i.e., the tag region) including the first and second imaging regions. The tag region is indicated by the dashed quadrangular frame in Fig. 9A. When the tag pulse is applied, the longitudinal magnetization of the blood in the first and second imaging regions and the background such as the myocardium is inverted and shifted to the negative side as shown in Fig. 9C.

After application of the tag pulse as shown in Fig. 9C, the fresh blood without being subjected to the tag pulse in the coronary artery flows into the first and second imaging regions from the upstream of the tag region. The blood flowing in from the upstream of the tag region is stronger in signal intensity than the background such as the myocardium or the blood in the tag region where the longitudinal magnetization has been inverted by the tag pulse. After elapse of the delay time TI, the blood in the coronary artery having strong signal intensity flows into the first and second imaging regions, and MR signals are acquired by the predetermined acquisition sequence.

The pulse sequence group repeats the pulse sequence shown in Fig. 9B, while changing its delay time TI for each time of repetition.

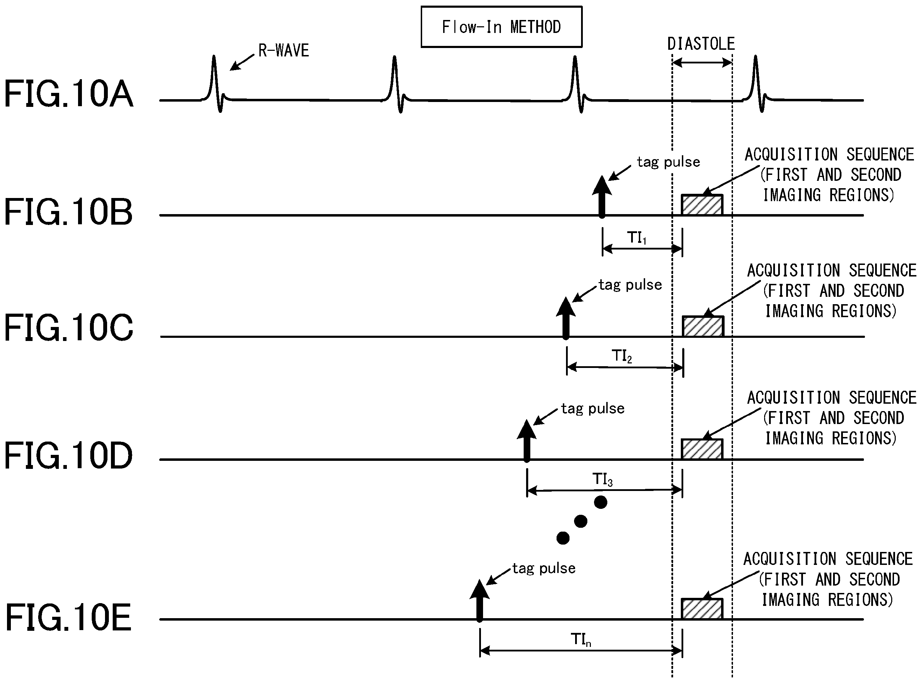

Fig. 10A to Fig. 10E illustrate the pulse sequence group of the Flow-In method based on the electrocardiographic synchronization imaging. Fig. 10A shows ECG signals for electrocardiographic synchronization, and Fig. 10B to Fig. 10E show respective sequence diagrams of the Flow-In method with different delay times TI.

The sequence diagrams of the Flow-In method shown in Fig. 10B to Fig. 10E are the same as those of the Flow-Out method shown in Fig. 8B to Fig. 8E except that the region non-selective IR pulse is not applied, and duplicate descriptions are omitted.

So far, a description has been given of the pulse sequence group set in the step ST100 in Fig. 4.

Returning to Fig. 4, in the step ST101 in Fig. 4, the pulse sequence group is applied to the object, and MR images including the first portion in the first imaging region and the second portion in the second imaging region are reconstructed for each of the different delay times TI. The processing of the step ST101 is performed by the reconstruction processing function F02 in Fig. 2.

In the next step ST102, the first perfusion curve is calculated from the pixel values for each delay time of the first portion of the coronary artery included in the first imaging region, and the second perfusion curve is calculated from the pixel values for each delay time of the second portion of the coronary artery included in the second imaging region, which is located downstream of the first imaging region. The processing of the step ST102 is performed by the perfusion-curve calculation function F03 in Fig. 2.

In the next step ST103, the first metrics (such as PH or TTP) are calculated from the first perfusion curve, and the second metrics (such as PH or TTP) are calculated from the second perfusion curve. The processing of the step ST103 is performed by the perfusion-metric calculation function F04 in Fig. 2.

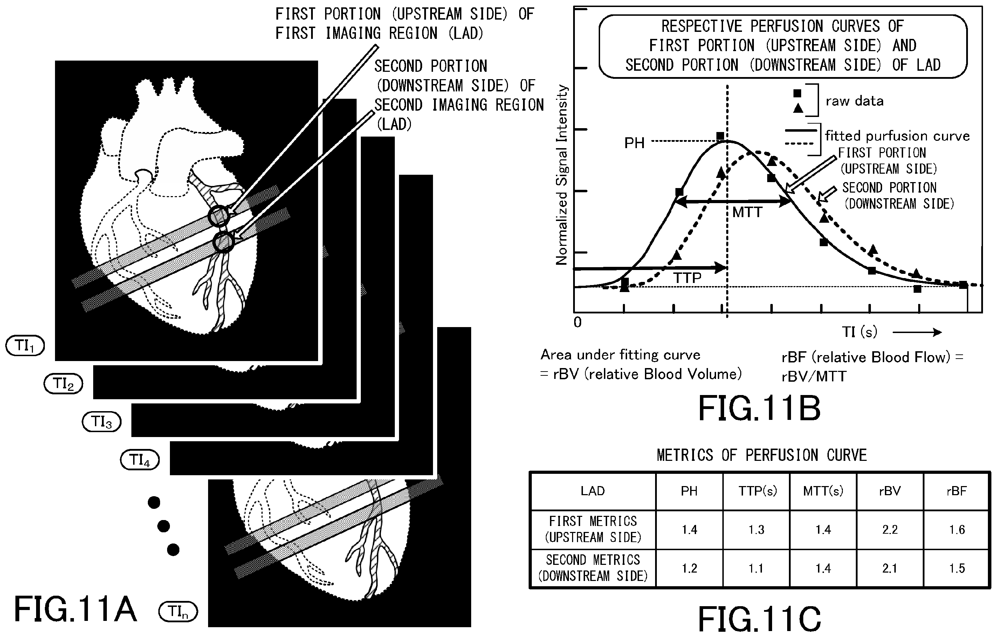

Fig. 11A to Fig. 11C illustrate the processing concept from the step ST101 to the step ST103.

Fig. 11A illustrates MR images for the respective delay times T1 generated in the step ST101. Fig. 11A schematically illustrates a total of n MR images from delay time TI₁ to delay time TIₙ. Regarding the left anterior descending artery (LAD) as the specific coronary artery, each of these MR images depicts the first portion (i.e., upstream side) of the LAD in the first imaging region and the second portion (i.e., downstream side) of the LAD in the second imaging region.

Fig. 11B is a graph illustrating the first and second perfusion curves generated in the step ST102. The horizontal axis of the graph indicates the delay time TI, and the vertical axis indicates normalized signal intensity. The black square dots in the graph indicate raw data of pixel values of the first portion (upstream side) of the LAD in the first imaging region in the MR image corresponding to each delay time TI. These raw data correspond to the first data of the coronary artery in the first imaging region described above.

Meanwhile, the black triangular dots in the graph indicate raw data of pixel values of the second portion (i.e., downstream side) of the LAD in the second imaging region in the MR image corresponding to each delay time TI. These raw data correspond to the second data of the coronary artery in the second imaging region described above.

The solid line in the graph indicates the first perfusion curve corresponding to the coronary artery in the first imaging region. When a portion of the coronary artery between the first imaging region and the second imaging region is defined as an evaluation target region, the first perfusion curve is a perfusion curve that shows the behavior of the blood flow in the immediate upstream portion of the evaluation target region. The first perfusion curve can be generated by curve-fitting the first data, which are the raw data in the MR image, with the use of a gamma variate function, for example.

On the other hand, tThe dashed line in the graph indicates the second perfusion curve corresponding to the coronary artery in the second imaging region. The second perfusion curve is a perfusion curve that shows the behavior of the blood flow in the immediate downstream portion of the evaluation target region. The second perfusion curve can be generated by curve-fitting the second data, which are the raw data in the MR image, with the use of a gamma variate function similarly to the first perfusion curve, for example.

Fig. 11C is a table illustrating the first metrics (on the upstream side) and the second metrics (on the downstream side) generated from the first and second perfusion curves in the step ST103. The first metrics (on the upstream side) and the second metrics (on the downstream side) are metrics calculated from the first and second perfusion curves. The metrics are PH (peak height) corresponding to the peak value of each perfusion curve, TTP (time to peak) corresponding to the delay time TI at which the perfusion curve reaches its peak, rBV (relative blood volume) corresponding to the area (i.e., square measure) of the region below the perfusion curve, MTT (mean transit time), and rBF (relative blood flow), for example.

Returning to Fig. 4, in the step ST104, occurrence/non-occurrence of stenosis or occlusion in the evaluation target region between the first and second portions of the coronary artery is evaluated by comparing the first and second perfusion curves in terms of shape or by comparing the first metrics with the second metrics.

The respective shapes of the first and second perfusion curves shown in Fig. 11B schematically show the shapes when there are no abnormal portions such as stenosis and occlusion in the evaluation target region of the coronary artery sandwiched between the first and second imaging regions. When there are no abnormal portion such as stenosis or occlusion in the evaluation target region of the coronary artery, the blood flow in the (upstream) first portion and the (downstream) second portion of the coronary artery above and below the evaluation target region each should show a similar behavior. Therefore, the shapes of the first perfusion curve (on the upstream side) and the second perfusion curve (on the downstream side) also each should show a similar shape. In addition, as shown in the table in Fig. 11C, it is considered that the first metrics (on the upstream side) are likely to show values similar to the respective values of the second metrics (on the downstream side).

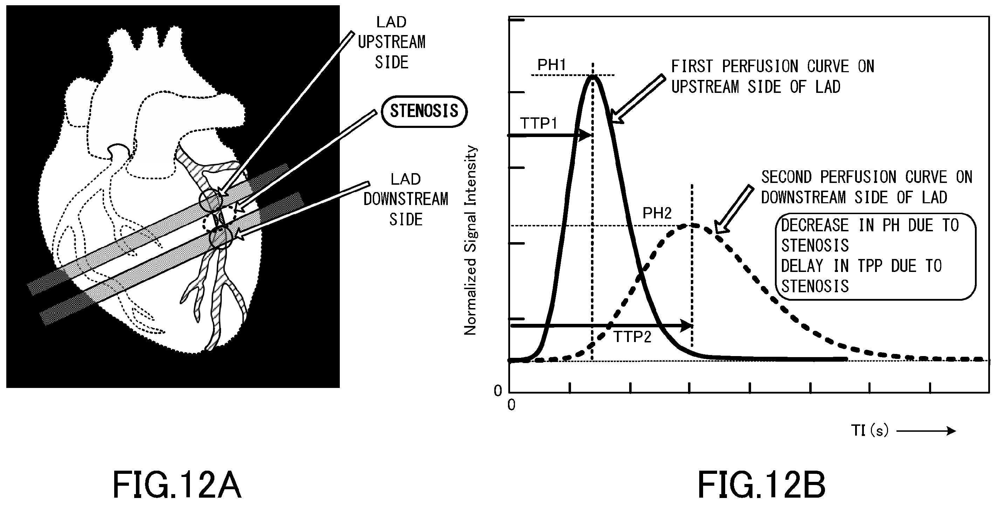

By contrast, Fig. 12B schematically shows respective shapes of the first perfusion curve (on the upstream side) and the second perfusion curve (on the downstream side) when there is an abnormal portion (Fig. 12A) such as stenosis or occlusion in the evaluation target region of the coronary artery sandwiched between the first and second imaging regions.

When there is an abnormal portion such as stenosis in the evaluation target region of the coronary artery as schematically shown in Fig. 12B, the second perfusion curve corresponding to the downstream side of the evaluation target region is considered to be lower in peak value PH than the first perfusion curve corresponding to the upstream side of the evaluation target region, and further, the time to peak TTP is considered to be delayed. Thus, in such a case, it can be evaluated that there is an abnormal portion such as stenosis between the first and second imaging regions.

As described above, each of the first and second imaging regions can be associated with each segment of the 17-segment model defined by the AHA. Thus, each of the first and second perfusion curves can also be associated with each segment of the 17-segment model. In this case, the first perfusion curve is associated with the upstream segment (i.e., the segment of the outer ring of the adjacent rings) in the group corresponding to the same coronary artery in the 17-segment model, and the second perfusion curve is associated with the downstream segment (i.e., the segment of the inner ring of the adjacent rings).

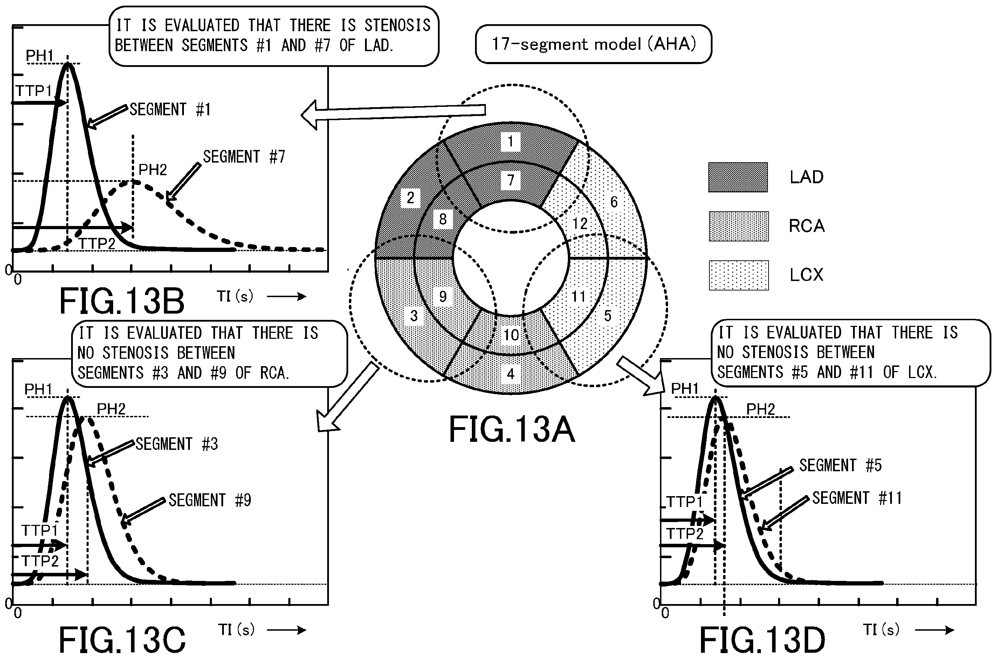

Fig. 13A to Fig. 13D illustrate the first and second perfusion curves associated with respective segments of the outermost and central rings of the 17-segment model. Fig. 13A shows the outermost ring and the central ring of the 17-segment model.

Fig. 13B illustrates the first perfusion curve corresponding to the segment #1 and the second perfusion curve corresponding to the segment #7. In this case, the peak height PH2 of the second perfusion curve is significantly lower than the peak height PH1 of the first perfusion curve, and the time to peak TTP2 of the second perfusion curve is much more delayed than the time to peak TTP1 of the first perfusion curve. Accordingly, for this case, it is evaluated that there is stenosis or there likely to be stenosis between the segments #1 and #7 of the left anterior descending artery (LAD) .

Note that, when occlusion occurs between the segments #1 and #7 of the left anterior descending artery (LAD) instead of stenosis, the peak height PH2 of the second perfusion curve is expected to become nearly zero.

Fig. 13C illustrates the first perfusion curve corresponding to the segment #3 and the second perfusion curve corresponding to the segment #9. In this case, the peak height PH2 of the second perfusion curve is approximately the same as the peak height PH1 of the first perfusion curve, and the time to peak TTP2 of the second perfusion curve is approximately the same as the time to peak TTP1 of the first perfusion curve. Accordingly, for this case, it is evaluated that there is no stenosis or there unlikely to be stenosis between the segments #3 and #9 of the right coronary artery (RCA) .

Similarly, Fig. 13D illustrates the first perfusion curve corresponding to the segment #5 and the second perfusion curve corresponding to the segment #11. Also in this case, the peak height PH2 of the second perfusion curve is approximately the same as the peak height PH1 of the first perfusion curve, and the time to peak TTP2 of the second perfusion curve is approximately the same as the time to peak TTP1 of the first perfusion curve. Accordingly, for this case as well, it is evaluated that there is no stenosis or there is unlikely to be stenosis between the segments #5 and #11 of the left circumflex artery (LCX).

Although similar evaluation can be performed between the segments #2 and #8, between the segments #4 and #10, and between the segments #6 and #12, those cases are not illustrated in the figure in order to avoid complication of the figure.

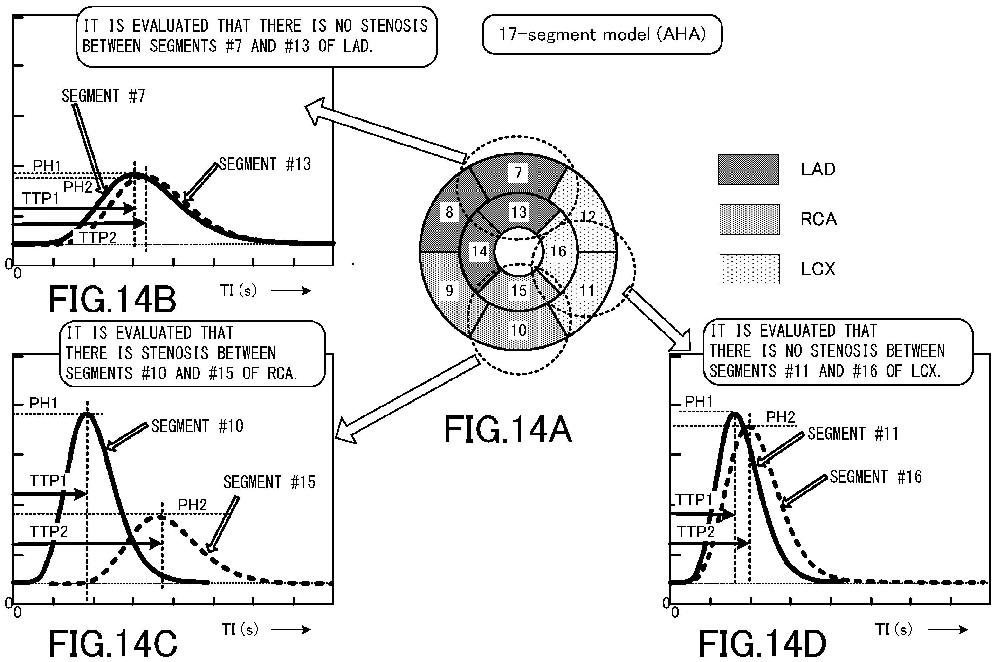

Fig. 14A to Fig. 14D show that the central ring and the innermost ring of the 17-segment model can be evaluated in a manner similar to the evaluation method shown in Fig. 13A to Fig. 13D.

In other words, it shows that presence/absence of an abnormal portion such as stenosis or occlusion can be evaluated by comparing shapes of the first and second perfusion curves and/or by comparing the first metrics with the second metrics between the segments on the upstream ring (central ring) and the downstream ring (innermost ring), each of which are associated with any one of the left anterior descending artery (LAD), the right coronary artery (RCA), and the left circumflex artery (LCX). In order to avoid duplication with the explanation of Fig. 13A to Fig. 13D, individual explanations of Fig. 14A to Fig. 14D will be omitted.

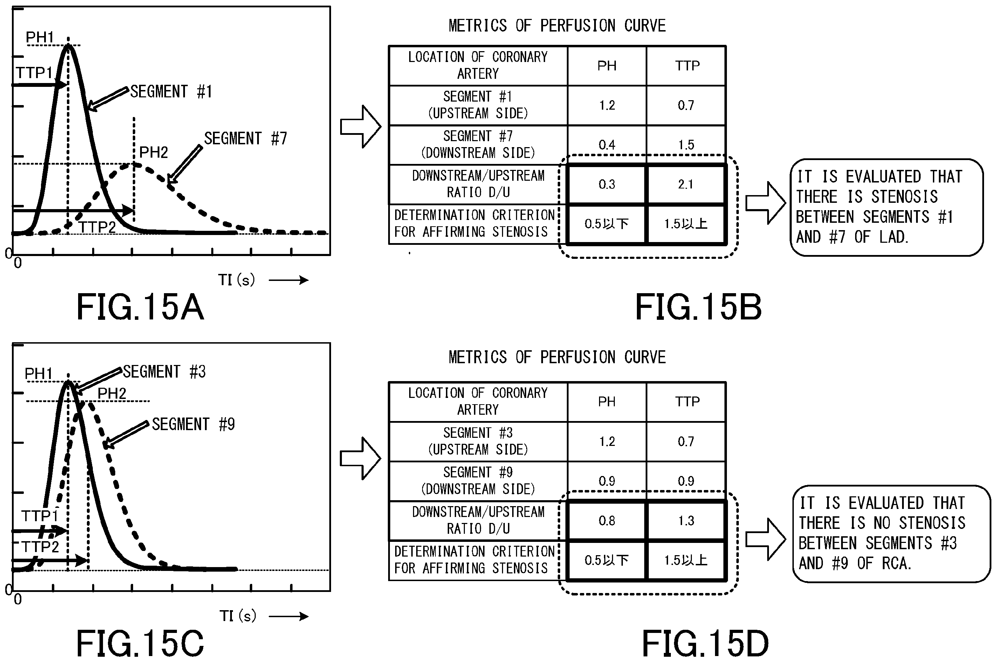

Fig. 15A to Fig. 15D are schematic diagrams illustrating a method of evaluating presence/absence of an abnormality such as stenosis or occlusion by using another evaluation metric other than the above-described metrics. Evaluation of presence/absence of an abnormal portion such as stenosis or occlusion in the evaluation target region between the first imaging region (on the upstream side) and the second imaging region (on the downstream side), or between the upstream segment and the downstream segment can also be performed by using the ratio of the second metric on the downstream side with respect to the first metric on the upstream side (hereafter referred to as the downstream/upstream ratio or D/U) .

Fig. 15A is a graph illustrating the first perfusion curve corresponding to the segment #1 and the second perfusion curve corresponding to the segment #7. Fig. 15B is a table illustrating a case of evaluating presence/absence of stenosis by using the downstream/upstream ratio (D/U).

The table in Fig. 15B shows the downstream/upstream ratio (D/U) and the evaluation criteria in addition to the first metrics (peak height PH and time to peak TTP of the segment #1) calculated from the first perfusion curve and the second metrics (peak height PH and time to peak TTP of the segment #7) calculated from the second perfusion curve. For example, if at least one of the downstream/upstream ratio (D/U) of the peak height PH and the downstream/upstream ratio (D/U) of the time to peak TTP satisfies the evaluation criterion, it is evaluated that there is stenosis.

In the case shown in Fig. 15B, the downstream/upstream ratio (D/U) of the peak height PH is 0.3, which satisfies the determination criterion of 0.5 or less. In addition, the downstream/upstream ratio (D/U) of the time to peak TTP is 2.1, which satisfies the determination criterion of 1.5 or more. Accordingly, evaluation for this case is that there is stenosis between the segments #1 and #7 of the left anterior descending artery (LAD).

Fig. 15C is a graph illustrating the first perfusion curve corresponding to the segment #3 and the second perfusion curve corresponding to the segment #9. Fig. 15D is a table illustrating how to evaluate presence/absence of stenosis between the segments #3 and #9 of the right coronary artery (RCA) by using the downstream/upstream ratio (D/U).

In the case shown in Fig. 15D, the downstream/upstream ratio (D/U) of the peak height PH is 0.8, which does not satisfy the determination criterion of 0.5 or less. Further, the downstream/upstream ratio (D/U) of the time to peak TTP is 1.3, which does not satisfy the determination criterion of 1.5 or more. Since both do not satisfy the corresponding determination criteria in this case, evaluation for this case is that there is no stenosis between the segments #3 and #9 of the right coronary artery (RCA).

So far, a description has been given of the evaluation of presence/absence of abnormalities of stenosis or occlusion in the step ST104 of Fig. 4. The various metrics, which is used in the evaluation in the step ST104, such as the shapes of the first and second perfusion curves, the first and second metrics calculated from the first and second perfusion curves, and the downstream/upstream ratio calculated from each of the first and second metrics are not strictly the same as the FFR, which is acquired in an examination using a catheter and/or guidewire. However, these various metrics can be considered as metrics that can be substituted for the FFR. Each of these metrics can be non-invasively acquired by using the MRI apparatus 1 of the embodiment without radiation exposure.

In the MRI apparatus 1 of the embodiment, the processing of the step ST105 may be additionally performed.

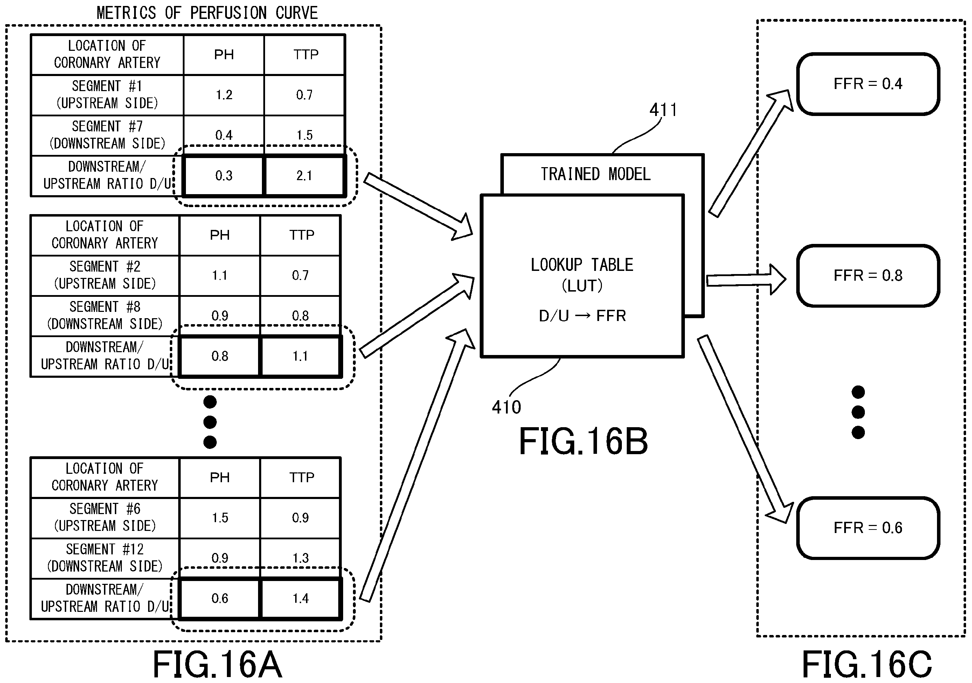

In the step ST105, from relationship between the first metrics and the second metrics , the FFR corresponding to the relationship is acquired by using a lookup table or a trained model. Fig. 16A to Fig. 16C are schematic diagrams illustrating a processing concept of the step ST105.

The lookup table 410 shown in Fig. 16B associates the relationship between the first metric and the second metric (for example, the downstream/upstream ratio (D/U)) with the fractional flow reserve (FFR), which is the ratio of the coronary-artery internal-pressure between the distal and proximal portions sandwiching the stenosis site of the coronary artery.
The lookup table 410 may be stored in the the memory 41.

Alternatively, a trained model may be used for associating the relationship between the first metric and the second metric with the FFR. The trained model 411 shown in Fig. 16B is a trained model that has been machine-learned so as to output the FFR in response to input of the relationship between the first metric and the second metric. The trained model 411 is also stored in the memory 41, for example.

Each table in Fig. 16A illustrates the first metrics calculated from the first perfusion curve, the second metrics calculated from the second perfusion curve, and metrics indicative of the relationship between the first and second metrics. Here, note that the first metrics are the PH or the TTP in the upstream segments #1, #2, and #6, for example, and the second metrics are the PH or the TTP in the downstream segments #7, #8, and #12, for example, and the metrics indicative of the relationship between the first and second metrics are the downstream/upstream ratios (D/U) in each table, for example.

The FFR associated with each metric can be acquired by using each of these metrics for referring to the lookup table 410. Alternatively, the FFR can be acquired from the trained model by inputting each of these metrics into the trained model.

The FFR acquired in the step ST105 can be considered to be a metric equivalent to the FFR acquired in an examination using a catheter and/or guidewire, and can be non-invasively acquired by using the MRI apparatus 1 of the embodiment without radiation exposure.

As described above, the second imaging region is the region located downstream of the first imaging region. The MRI apparatus 1 of the embodiment sets the pulse sequence group that acquires both the first data for generating the first perfusion data from the behavior of the blood flow in the coronary artery of the object included in the first imaging region and the second data for generating the second perfusion data from the behavior of the blood flow in the coronary artery included in the second imaging region located downstream of the first imaging region.

The MRI apparatus 1 of the embodiment compares the first and second perfusion data, both of which are generated by applying the pulse sequence group to the object, and evaluates occurrence/non-occurrence of stenosis or occlusion between the first portion of the coronary artery included in the first imaging region and the second portion of the coronary artery included in the second imaging region.

When of applying the above-described processing of the MRI apparatus 1 to the 17-segment model based on the AHA classification, the first imaging region can be associated with one segment in the outermost ring (i.e., Basal ring) of the three rings of the 17-segment model, and the second imaging region can be associated with one segment in the central ring (i.e., Mid-Cavity ring) of the three rings. Alternatively, the first imaging region can be associated with one segment in the central ring (i.e., Mid-Cavity ring) of the three rings of the 17-segment model, and the second imaging region can be associated with one segment in the innermost ring (i.e., Apical ring) of the three rings.

Note that the three rings of the 17-segment model, i.e., Basal ring, Mid-Cavity ring, and Apical ring, can be associated with the first imaging region, the second imaging region, and the third imaging region, respectively. In this case, the first, second, and third data respectively corresponding to Basal ring, Mid-Cavity ring, and Apical ring as the imaging regions may be acquired from these three imaging regions in advance by using a second pulse sequence group, for example. The second pulse sequence group may be substantially the same as the pulse sequence group as shown in Fig. 8A to Fig. 8E or in Fig. Fig. 10A to Fig. 10E.

In the case of evaluating the stenosis or occlusion between adjacent rings, for example, Basal ring and Mid-Cavity ring, it can be achieved by generating the first perfusion data and the second perfusion data (or the first perfusion curve and the second perfusion curve) from the first data and the second data, and comparing both. For example, in the case of evaluating stenosis or occlusion between Mid-Cavity ring and Apical ring, it can be achieved by generating the second perfusion data and the third perfusion data (or the second perfusion curve and the third perfusion curve) from the second data and the third data, and then comparing both.

In this case, when evaluating stenosis or occlusion between Basal ring and Mid-Cavity ring, the explanation of the above-mentioned embodiment can be read as it is.

On the other hand, when evaluating stenosis or occlusion between Mid-Cavity ring and Apical ring, "the first imaging region" can be replaced with "the second imaging region", and "the second imaging region" in the description of the above-mentioned embodiment can be replaced with the third imaging region.

Similarly, it is sufficient to interpret the above description such that the first perfusion data are replaced by the second perfusion data and the second perfusion data are replaced by the third perfusion data. Similarly, it is sufficient to interpret the above description such that the first perfusion curve is replaced by the second perfusion curve and the second perfusion curve is replaced by the third perfusion curve. Similarly, the first data are replaced by the second data, and the second data are replaced by the third data. Similarly, the first metrics are replaced by the second metrics, and the second metrics are replaced by the third metrics. Similarly, the first portion is replaced by the second portion, and the second portion is replaced by the third portion.

The display control function F06 may display at least one of the perfusion curves (e.g., the first, second, and third perfusion curves) and the perfusion-related metrics (e.g., the first, second, and third metrics) corresponding to the respective imaging regions in such a manner that each displayed item is associated with the corresponding segment of the 17-segment model. Of the displayed 17-segment model, the perfusion-related metric(s) and the perfusion curve(s) corresponding to the segment selected by a user operation via the input I/F 43 may be displayed.

Note that the inflow path included in each of the imaging regions is not limited to the coronary artery described above but may also be another inflow vessel such as the hepatic artery and the internal carotid artery. Furthermore, the inflow path may also be a body cavity, such as a subarachnoid space, i.e., space within the body. The body fluid in the inflow path is not limited to the blood described above but may also be other body fluid such as cerebrospinal fluid (CSF). The perfusion data and/or the perfusion curves can be generated from the behavior of the blood flow, as well as from the behavior of the body fluid flow. In other words, the perfusion data and/or the perfusion curves may be attributable not only to blood but also to other body fluid.

A disease to be evaluated by comparison of a plurality of perfusion data sets are not limited to a disease within the inflow path such as the above-described stenosis and occlusion. A disease to be evaluated by comparison of a plurality of perfusion data sets may be a disease that is capable of affecting an inflow path or is located in the vicinity of the inflow path, such as myocardial infarction.

According to the MRI apparatus of each embodiment described above, a metric for evaluating the presence or absence of a disease in an inflow path of an object such as a metric equivalent to or substitutable for the FFR can be acquired by using the MRI apparatus without involving radiation exposure and without performing an invasive examination using a catheter and/or guidewire.

Note that the imaging-condition setting function F01 in the description of the embodiment is an example of a setting unit. The reconstruction processing function F02 and the perfusion-curve calculation function F03 in the description of the embodiment are examples of a generation unit. The perfusion-metric calculation function F04 in the description of the embodiment is an example of a calculation unit. The evaluation function F05 in the description of the embodiment is an example of an evaluation unit. The display control function F06 in the description of the embodiment is an example of a display control unit.

While certain embodiments have been described, these embodiments have been presented by way of example only, and are not intended to limit the scope of the invention. Indeed, the novel methods and systems described therein may be embodied in a variety of other forms; furthermore, various omissions, substitutions and changes in the form of the methods and systems described therein may be made without departing from the scope of the invention as defined by the appended claims. The accompanying claims are intended to cover such forms or modifications as would fall within the scope of the invention.

## Claims

1. An MRI apparatus comprising:
a setting unit configured to set a pulse sequence group that acquires first data and second data,
the first date being for generating first perfusion data from behavior of a body fluid of an inflow path of an object included in a first imaging region,
the second data being for generating second perfusion data from behavior of a body fluid of the inflow path included in a second imaging region located downstream of the first imaging region;
a generation unit configured to generate the first perfusion data and the second perfusion data, both of which are obtained by applying the pulse sequence group to the object; and
an evaluation unit configured to evaluate a disease status by comparing the first perfusion data with the second perfusion data.

2. The MRI apparatus according to claim 1, wherein:
the inflow path is an inflow blood vessel,
the body fluid is blood, and
the evaluation unit is configured to evaluate whether stenosis or occlusion has occurred between a first portion of the flow path included in the first imaging region and a second portion of the flow path included in the second imaging region.

3. The MRI apparatus according to claim 1 or 2, wherein:
the setting unit is further configured to set a second pulse sequence group that acquires;
the first data for generating the first perfusion data from behavior of the body fluid of the inflow path of the object included in the first imaging region,
the second data for generating the second perfusion data from behavior of the body fluid of the inflow path included in the second imaging region located downstream of the first imaging region, and
third data for generating third perfusion data from behavior of the body fluid of the inflow path included in a third imaging region located further downstream of the second imaging region,
the evaluation unit is further configured to;
(a) evaluate the disease status by comparing the first perfusion data and the second perfusion data, both of which are generated by applying the second pulse sequence group to the object, or
(b) evaluate the disease status by comparing the second perfusion data and the third perfusion data, both of which are generated by applying the second pulse sequence group to the object.

4. The MRI apparatus according to claim 3, wherein:
the inflow path is an inflow blood vessel,
the body fluid is blood, and
the evaluation unit is further configured to;
(a) evaluate whether stenosis or occlusion has occurred between the first portion of the inflow path included in the first imaging region and the second portion of the inflow path included in the second imaging region by comparing the first perfusion data and the second perfusion data, both of which are generated by applying the second pulse sequence group to the object, or
(b) evaluate whether stenosis or occlusion has occurred between the second portion of the inflow path included in the second imaging region and a third portion of the inflow path included in the third imaging region by comparing the second perfusion data and the third perfusion data, both of which are generated by applying the second pulse sequence group to the object.

5. The MRI apparatus according to any one of claims 1 to 4, wherein each of the pulse sequence group and the second pulse sequence group set by the setting unit is a pulse sequence group in which behavior of body fluid of the inflow path is imaged without using a contrast agent.

6. The MRI apparatus according to any one of claims 1, 2, and 5, wherein the inflow path is coronary artery, and the setting unit is configured to set the pulse sequence group in such a manner that:
each of the first imaging region and the second imaging region is provided approximately in parallel with a short-axis cross-section of a heart and has a predetermined thickness in a long-axis direction of the heart; and
the first imaging region is set at a position closer to Basal than the second imaging region.

7. The MRI apparatus according to any one of claims 3 to 5, wherein the inflow path is coronary artery, and the setting unit is configured to set the second pulse sequence group in such a manner that:
each of the first imaging region, the second imaging region, and the third imaging region is provided approximately in parallel with a short-axis cross-section of a heart and has a predetermined thickness in a long-axis direction of the heart;
the first imaging region is set closer to Basal than the second imaging region; and
the third imaging region is set closer to Apical than the second imaging region.

8. The MRI apparatus according to any one of claims 1 to 6,
wherein each of the first imaging region and the second imaging region corresponds to at least one segment that is obtained by dividing the heart in accordance with a Bull's Eye 17-segment model based on classification by American Heart Association (AHA),
wherein the first imaging region and the second imaging region are set in such a manner that:
(a) when the first imaging region is a segment in an outermost ring of three rings of the 17-segment model, the second imaging region is one segment in a central ring of the three rings and is a segment in a same radial direction as the first imaging region; or
(b) when the first imaging region is one segment in the central ring of the three rings of the 17-segment model, the second imaging region is one segment in an innermost ring of the three rings and is a segment in a same radial direction as the first imaging region.

9. The MRI apparatus according to claim 8, wherein:
the 17-segment model has a total of 17 segments composed of segments #1 to #17 classified by the AHA; and
when the first imaging region is a segment #1, #2, #3, #4, #5, or #6, the second imaging region is a region corresponding to a segment #7, #8, #9, #10, #11, or #12, respectively.

10. The MRI apparatus according to claim 8, wherein:
the 17-segment model has a total of 17 segments composed of segments #1 to #17 classified by the AHA;
when the first imaging region is a segment #7, the second imaging region a region corresponding to a segment #13;
when the first imaging region is a segment #8, the second imaging region is a region corresponding to a segment #13 or #14;
when the first imaging region is a segment #9, the second imaging region is a region corresponding to a segment #14 or #15;
when the first imaging region is a segment #10, the second imaging region is a region corresponding to a segment #15;
when the first imaging region is a segment #11, the second imaging region is a region corresponding to a segment #15 or #16; and
when the first imaging region is a segment #12, the second imaging region is a region corresponding to a segment #16 or #13.

11. The MRI apparatus according to any one of claims 1 to 10, further comprising a display control unit,
wherein each of the first imaging region and the second imaging region corresponds to at least one segment that is obtained by dividing the heart in accordance with a Bull's Eye 17-segment model based on classification by American Heart Association (AHA),
the display control unit is configured to display at least one of perfusion-related metrics and perfusion curves in association with with the at least one segment.

12. The MRI apparatus according to any one of claims 1 to 11, wherein:
the pulse sequence group includes a plurality of pulse sequences having a tag pulse for labeling body fluid; and
each of the plurality of pulse sequences is a pulse sequence group that acquires the first data of the inflow path in the first imaging region and the second data of the inflow path in the second imaging region by delaying a data acquisition timing from application of the tag pulse by a delay time TI that is set to be different for each of the plurality of pulse sequences.

13. The MRI apparatus according to any one of claims 1 to 11, wherein:
the pulse sequence group corresponds to a time-SLIP Flow-Out method in which a tag pulse is applied to a tag region provided upstream of the first imaging region and then the first data and the second data are respectively acquired from the first imaging region and the second imaging region by delaying a data acquisition timing from application of the tag pulse by a plurality of different delay times TI; and
the generation unit is configured to
generate the first perfusion data from acquired first data and
generate the second perfusion data from acquired second data.

14. The MRI apparatus according to any one of claims 1 to 11, wherein:
the pulse sequence group corresponds to a time-SLIP Flow-In method in which a tag pulse is applied to a region including the first imaging region and the second imaging region and then the first data and the second data are respectively acquired from the first imaging region and the second imaging region by delaying a data acquisition timing from application of the tag pulse by a plurality of different delay times TI; and
the generation unit is configured to
generate the first perfusion data from acquired first data and
generate the second perfusion data from acquired second data.

15. The MRI apparatus according to any one of claims 1 to 14, wherein the pulse sequence group set by the setting unit is a pulse sequence group in which behavior of blood in the coronary artery is imaged by administering a contrast agent.
